# EUROPEAN PATENT APPLICATION

(11) **EP 4 534 691 A1**
(43) Date of publication of application: **09.04.2025**
(21) Application number: 22943186.1
(22) Date of filing: 27.05.2022
(51) Int. Cl.: C12Q 1/6869

(54) **METHOD FOR MAINTAINING NANOPORE SEQUENCING SPEED**

(71) Applicant: BGI Shenzhen, Shenzhen, Guangdong 518083 (CN); BGI Shenzhen Co., Ltd, Shenzhen, Guangdong 518083 (CN)
(72) Inventor: JI, Zhouxiang, Shenzhen, Guangdong 518083 (CN); LU, Jingxiong, Shenzhen, Guangdong 518083 (CN); ZHENG, Rongrong, Shenzhen, Guangdong 518083 (CN); ZHANG, Wenwei, Shenzhen, Guangdong 518083 (CN); DONG, Yuliang, Shenzhen, Guangdong 518083 (CN); LI, Yuxiang, Shenzhen, Guangdong 518083 (CN); ZENG, Tao, Shenzhen, Guangdong 518083 (CN); XU, Xun, Shenzhen, Guangdong 518083 (CN)
(74) Representative: Ström & Gulliksson AB
(86) International application number: PCT/CN2022/095500
(87) International publication number: WO 2023/225988

(57) **Abstract**

Provided in the present disclosure is a method for sequencing a double-stranded target polynucleotide. The method can keep ATP at a relatively constant concentration during sequencing, so that the sequencing rate can be better kept stable and unchanged. Further provided in the present disclosure is a kit for sequencing a double-stranded target polynucleotide, the kit including a transmembrane pore in the membrane, a helicase, an ATP-generating enzyme and an ATP-generating substrate.

## Description

### FIELD

The present disclosure relates to a method for maintaining nanopore sequencing speed, and further provides a method for sequencing a double-stranded target polynucleotide and a kit for sequencing a double-stranded target polynucleotide.

### BACKGROUND

With the continuous development of nanotechnology, the nanopore sequencing technology that can realize nucleic acid sequence reading at single molecule level has developed rapidly. This technology has obvious advantages compared with the next generation sequencing technology in terms of portability, sequencing read length and sequencing speed, etc. In 1996, scientists used the alpha-hemolysin protein to identify different bases for the first time (Kasianowicz, John J., et al. Proceedings of the National Academy of Sciences 93.24

(1996): 13770-13773.), which pioneered DNA sequencing based on the principle of nanopore detection.

British Oxford Nanopore Technologies has launched a number of sequencers with different throughputs. The principle is that: transmembrane proteins with a diameter of nanometer scale are inserted into a polymer membrane to form a nanopore channel on the membrane. Two electrodes are placed on both sides of the membrane, and conductive buffer is placed on both sides of the membrane. After electrified, ions generate current through the transmembrane proteins. The double-stranded DNA of a sequencing library is unwound into single-stranded DNA under the action of helicase, and the single-stranded DNA passes through the transmembrane protein under the action of electric field force to produce the characteristic current signals. The characteristic current signals are analyzed through an algorithm to realize sequence reading. Wherein the helicase uses the energy generated by the hydrolysis of adenosine triphosphate (ATP) or guanosine triphosphate (GTP) to move along a nucleic acid skeleton in a specific direction (5'-3' or 3'-5') to unwind hydrogen-bonded double-stranded DNA into single-stranded DNA.

### SUMMARY

The inventors of the present disclosure find that there is a problem in the above sequencing process. That is, as the sequencing progresses, the sequencing speed of the nanopore decreased. The decrease of the sequencing speed will affect sequencing throughput and sequencing accuracy. Therefore, to consistently maintain high throughput and high accuracy during the sequencing process, a method that can maintain nanopore sequencing speed shall be provided.

In order to solve the above problem, the inventors of the present disclosure found through numerous experiments that the nanopore sequencing speed is related to the composition of conductive buffer on one side or both sides of the membrane. Further, through numerous experiments and repeated explorations, the inventors of the present disclosure realized for the first time that controlling the amounts of ADP and ATP at the same time in the above sequencing process is the key to affecting the sequencing rate, and thus completed the present disclosure.

Therefore, the present disclosure provides a method for sequencing a double-stranded target polynucleotide, including:
(a) providing a double-stranded target polynucleotide, a membrane containing a transmembrane pore, a helicase, an ATP-generating enzyme and an ATP-generating substrate, wherein the ATP-generating substrate is capable of reacting with ADP to generate ATP under the catalysis of the ATP-generating enzyme;
(b) contacting the double-stranded target polynucleotide with the transmembrane pore, the helicase, the ATP-generating enzyme and the ATP-generating substrate, wherein the double strands of the double-stranded target polynucleotide are separated by the helicase to form a single-stranded target polynucleotide, and wherein the helicase enables the single-stranded polynucleotide to move in the transmembrane pore, allowing a portion of nucleotides in the single-stranded polynucleotide to interact with the transmembrane pore;
(c) measuring current that passes through the transmembrane pore during each interaction to determine the sequence of the double-stranded target polynucleotide.

In the method herein, different nucleotides (e.g., A, T, C and G) have different effects on the current passing through the transmembrane pore, and the transmembrane pore can distinguish the nucleotides with similar structures. Thus, in some embodiments, individual nucleotides can be identified at the single molecule level according to the amplitude of current or the duration of the interaction when each nucleotide interacts with the transmembrane pore. If a characteristic current associated with the nucleotide is detected flowing through the pore, then the type (e.g., A, T, C and G) of the nucleotide passing through the pore can be determined, that is, the sequencing is realized. By continuously identifying the nucleotides in the target polynucleotide, the sequence of the target polynucleotide can be estimated or determined.

In some embodiments, the method further includes measuring the current passing through the pore during the interaction with the nucleotide. Therefore, in some embodiments, the method also provides a circuit capable of applying an electric potential and measuring an electrical signal across the membrane and the pore. In some embodiments, the method also provides a patch clamp or voltage clamp.

In the method herein, the double strands of the double-stranded target polynucleotide are separated by the helicase to form the single-stranded target polynucleotide. In some embodiments, the helicase uses the energy generated by the hydrolysis of adenosine triphosphate (ATP) or guanosine triphosphate (GTP) to move along a nucleic acid skeleton in a specific direction (5'-3' or 3'-5') to unwind the hydrogen-bonded double-stranded DNA into single-stranded DNA.

In some embodiments, the helicase controls the movement of the single-stranded polynucleotide along the field generated by the applied voltage to pass through the pore. In some embodiments, the helicase acts as a brake, preventing the single-stranded polynucleotide from moving through the pore too quickly under the influence of the applied voltage. In some embodiments, the method further includes: (d) reducing the voltage applied across the pore so that the single-stranded polynucleotide moves through the pore in a direction opposite to that in step (b), and a portion of nucleotides in the polynucleotide interact with the pore to measure the current passing through the pore during each interaction, thereby performing a correction read on the sequence of the target polynucleotide obtained in step (c).

In some embodiments, the single-stranded polynucleotide may interact with the pore on either side of the membrane. The single-stranded polynucleotide may interact with the pore at any position in any manner.

In some embodiments, the ATP-generating enzyme is selected from: pyruvate kinase, acetate kinase, creatine phosphokinase, serine kinase, threonine kinase, tyrosine kinase, FoF1-ATPase, polyphosphate kinase, nucleoside diphosphate kinase, or any combination thereof.

In some embodiments, the ATP-generating enzyme is a pyruvate kinase, and the ATP-generating substrate is phosphoenolpyruvate. In some embodiments, the phosphoenolpyruvate is 5 mM and the pyruvate kinase is 0.02 U/mL. In some embodiments, the phosphoenolpyruvate is 5 mM and the pyruvate kinase is 0.2 U/mL. In some embodiments, the phosphoenolpyruvate is 5 mM and the pyruvate kinase is 2 U/mL.

In some embodiments, the ATP-generating enzyme is the acetate kinase, and the ATP-generating substrate is lithium potassium acetyl phosphate. In some embodiments, the lithium potassium acetyl phosphate is 5 mM and the acetate kinase is 0.02 U/mL.

In some embodiments, the ATP-generating enzyme is the creatine phosphokinase, and the ATP-generating substrate is creatine phosphate disodium.

In some embodiments, the ATP-generating enzyme is the FoF1-ATPase, and the ATP-generating substrate is inorganic phosphate.

In some embodiments, the ATP-generating enzyme is the creatine phosphokinase, and the ATP-generating substrate is creatine phosphate.

In some embodiments, the ATP-generating enzyme is the polyphosphate kinase, and the ATP-generating substrate is polyphosphate.

In some embodiments, the ATP-generating enzyme is the nucleoside diphosphate kinase, and the ATP-generating substrate is nucleoside triphosphate.

Herein, the amounts of the ATP-generating enzyme and the ATP-generating substrate used herein are not limited to the specific concentrations and amounts used in the embodiments, as long as the concentration of ATP can be kept relatively constant during the sequencing process so that the sequencing rate can be better kept stable and unchanged. In fact, during the sequencing process, under the action of the ATP-generating enzyme, the substrate will react and the amount will be gradually decreased. Those skilled in the art have the ability to properly adjust the concentrations and the amounts of the ATP-generating enzyme and the ATP-generating substrate in a sequencing system according to an experimental purpose, to obtain appropriate concentrations and amounts of the ATP-generating enzyme and the ATP-generating substrate.

In some embodiments, the helicase is selected from Dda, UvrD, Rep, RecQ, PcrA, eIF4A, NS3, Rep, gp41, T7gp4, or any combination thereof.

In some embodiments, the helicase is further linked to an additional polypeptide, the additional polypeptide being selected from a label, an enzyme cleavage site, a signal peptide or leading peptide, a detectable marker, or any combination thereof.

In some embodiments, the helicase is a wild type Dda or a mutant thereof.

In some embodiments, the helicase has an amino acid sequence as set forth in SEQ ID NO: 2.

In some embodiments, the transmembrane pore is a transmembrane protein pore or a transmembrane solid-state pore.

In some embodiments, the transmembrane protein pore is selected from hemolysin, MspA, MspB, MspC, MspD, Frac, ClyA, PA63, CsgG, CsgD, XcpQ, SP1, phi29 connector, T7 connector, GspD, InvG, or any combination thereof.

In some embodiments, the transmembrane pore is further linked to an additional polypeptide, the additional polypeptide being selected from a label, an enzyme cleavage site, a signal peptide or leading peptide, a detectable marker, or any combination thereof.

In some embodiments, the transmembrane pore is a wild type CsgG protein or a mutant thereof. In some embodiments, the transmembrane pore has an amino acid sequence as set forth in SEQ ID NO: 1.

In some embodiments, the membrane is an amphiphilic layer (e.g., phospholipid bilayer) or a polymer membrane (e.g., di-block and tri-block).

The method described above can be performed using any suitable membrane. In some embodiments, the membrane is a phospholipid bilayer, wherein the transmembrane pore is inserted into the phospholipid bilayer.

In some embodiments, the method is generally performed using the following membrane that is: (i) an artificial bilayer containing pores, (ii) an isolated naturally-occurring lipid bilayer containing pores, or (iii) a cell having pores inserted therein. The method is preferably performed using an artificial bilayer (e.g., an artificial phospholipid bilayer).

In some embodiments, the double-stranded target polynucleotide is a double-stranded DNA and/or a double-stranded DNA-RNA hybrid.

In some embodiments, the double-stranded target polynucleotide is naturally occurring and/or artificially synthesized.

In some embodiments, the double-stranded target polynucleotide is obtained from biological samples extracted from viruses, prokaryotes (e.g., bacteria), eukaryotes (e.g., plants (e.g., cereals, legumes, fruits or vegetables) ), mammals (e.g., horses, cattle, mice or humans), or any combination thereof.

In some embodiments, the double strands of the double-stranded target polynucleotide are linked by a bridging part at or near one end of the target polynucleotide; and the bridging part is selected from a polymer linker, a chemical linker, a polynucleotide or a polypeptide.

In some embodiments, the double-stranded target polynucleotide is circular or linear.

In some embodiments, all or only a portion of the double-stranded target polynucleotide may be sequenced using the method described above. The double-stranded target polynucleotide may be of any length. For example, the double-stranded target polynucleotide may be at least 10, at least 50, at least 100, at least 150, at least 200, at least 250, at least 300, at least 400, or at least 500 nucleotide pairs in length. The double-stranded target polynucleotide may be 1,000 or more nucleotide pairs, 5,000 or more nucleotide pairs, or 100,000 or more nucleotide pairs in length. The double-stranded target polynucleotides may be naturally occurring or artificially synthesized. For example, the method can be used to verify the sequence of an artificially synthesized oligonucleotides, and the method is usually performed in vitro.

In some embodiments, the double-stranded target polynucleotide includes at least one single-stranded overhang (e.g., a 5' overhang and/or a 3' overhang); the single-stranded overhang includes a leader sequence; and the leader sequence guides a nucleic acid strand linked thereto into the pore.

In some embodiments, in step (b), the double-stranded target polynucleotide is contacted with the helicase to form a complex, and then the complex is contacted with the transmembrane pore.

In some embodiments, the method described above is generally performed in the presence of a reagent for nanopore sequencing.

In some embodiments, in step (b), the reagent for nanopore sequencing is contacted with the helicase.

In some embodiments, the reagent for nanopore sequencing is selected from ATP, inorganic salts (e.g., chloride salt, e.g., sodium chloride, potassium chloride or lithium chloride), buffers (HEPES and/or Tris-HCl), EDTA, metal ions (e.g., Mn²⁺, Mg²⁺, Co⁺, Zn²⁺, Cu²⁺, Cu⁺, Ni⁺, Fe²⁺ or Fe³⁺), or any combination thereof.

In some embodiments, the concentration of the chloride salt is saturated.

In some embodiments, the concentration of the chloride salt is 0.1 M to 2.5 M, 0.3 M to 1.9 M, 0.5 M to 1.8 M, 0.7 M to 1.7 M, 0.9 M to 1.6 M or 1 M to 1.4 M.

In some embodiments, the method described above is performed at a pH of 4.0 to 12.0, 4.5 to 10.0, 5.0 to 9.0, 5.5 to 8.8, 6.0 to 8.7, or 7.0 to 8.8, or 7.5 to 8.5. In some embodiments, the method described above is performed at a pH of 7.5.

In another aspect, the present disclosure provides a kit, including: a membrane containing a transmembrane pore, a helicase, an ATP-generating enzyme and an ATP-generating substrate; and optionally, the kit further includes a reagent for nanopore sequencing.

In some embodiments, the reagent for nanopore sequencing is selected from ATP, inorganic salts (e.g., chloride salt, e.g., sodium chloride, potassium chloride or lithium chloride), buffers (HEPES and/or Tris-HCl), EDTA, metal ions (e.g., Mn²⁺, Mg²⁺, Co⁺, Zn²⁺, Cu²⁺, Cu⁺, Ni⁺, Fe²⁺ or Fe³⁺), or any combination thereof.

In some embodiments, the ATP-generating enzyme is selected from: pyruvate kinase, acetate kinase, creatine phosphokinase, serine kinase, threonine kinase, tyrosine kinase, FoF1-ATPase, polyphosphate kinase, nucleoside diphosphate kinase, or any combination thereof.

In some embodiments, the kit includes: a membrane containing a transmembrane pore; a helicase; an ATP; (I) pyruvate kinase and phosphoenolpyruvate, (II) acetate kinase and lithium potassium acetyl phosphate, (III) creatine phosphokinase and creatine phosphate, (IV) FoF1-ATPase and inorganic phosphate; (V) creatine phosphokinase and creatine phosphate; (VI) polyphokinase and polyphosphate; (VII) nucleoside diphosphate kinase and nucleoside triphosphate, or any combination of (I) to (VII).

In some embodiments, the kit is used to sequence the double-stranded target polynucleotide.

In some embodiments, the helicase is selected from Dda, UvrD, Rep, RecQ, PcrA, eIF4A, NS3, Rep, gp41, T7gp4, or any combination thereof.

In some embodiments, the helicase is further linked to an additional polypeptide, the additional polypeptide being selected from a label, an enzyme cleavage site, a signal peptide or leading peptide, a detectable marker, or any combination thereof.

In some embodiments, the helicase is a wild type Dda or a mutant thereof.

In some embodiments, the helicase has an amino acid sequence as set forth in SEQ ID NO: 2.

In some embodiments, the transmembrane pore is a transmembrane protein pore or a transmembrane solid-state pore. In some embodiments, the transmembrane protein pore is selected from hemolysin, MspA, MspB, MspC, MspD, Frac, ClyA, PA63, CsgG, CsgD, XcpQ, SP1, phi29 connector, T7 connector, GspD, InvG, or any combination thereof.

In some embodiments, the transmembrane pore is further linked to an additional polypeptide, the additional polypeptide being selected from a label, an enzyme cleavage site, a signal peptide or leading peptide, a detectable marker, or any combination thereof.

In some embodiments, the transmembrane pore is a wild type CsgG protein or a mutant thereof. In some embodiments, the transmembrane pore has an amino acid sequence as set forth in SEQ ID NO: 1.

In some embodiments, the membrane is an amphiphilic layer (e.g., phospholipid bilayer) or a polymer membrane (e.g., di-block and tri-block).

Use of the above kit in the preparation of a sequencing device is provided for sequencing the double-stranded target polynucleotide.

### Definition of terms

Unless defined otherwise, all technical and scientific terms used herein have the same meanings as those commonly understood by those ordinary skilled in the art to which the present disclosure belongs. All patents, applications and other publications mentioned herein are hereby incorporated by reference in their entirety. In the event of a conflict or inconsistency between the definitions set forth herein and the definitions set forth in patents, applications, and other publications incorporated herein by reference, the definitions set forth herein shall control.

As used herein, the term "polynucleotide" is a macromolecule containing one, two or more than two nucleotides. The polynucleotide can include any combination of any nucleotide, which may be naturally occurring or artificial. The nucleotide generally contains a nucleobase, a sugar and at least one phosphate group.

As used herein, the term "transmembrane pore" is a structure that allows hydrated ions driven by an applied electric potential to flow from one side of the membrane to the other side of the membrane, and the transmembrane pore is generally inserted into the membrane (e.g., lipid bilayer). The transmembrane pore is preferably a transmembrane protein pore. The "transmembrane protein pore" is a polypeptide or a collection of polypeptides that allows hydrated ions to flow from one side of the membrane to the other side of the membrane. The transmembrane protein pore allows the polynucleotide to move and pass through the pore. The transmembrane protein pore generally includes a barrel or channel through which ions can flow. The transmembrane protein pore generally includes amino acids that facilitate the interaction with a target nucleotide, and these amino acids are preferably located near a constriction of the barrel or channel.

As used herein, the term "movement/move" is to make the single-stranded polynucleotide move from one side of the pore to the other side. The movement of the single-stranded polynucleotide through the pore may be influenced by electropotential action or enzymatic action, as well as electropotential and enzymatic action. The movement may be unidirectional or bidirectional.

As used herein, the term "ATP-generating enzyme" refers to an enzyme that can catalyze the substrates in a system to generate ATP.

As used herein, the term "ATP-generating substrate" refers to a substance that can convert or generate ATP in the presence of the ATP-generating enzyme. In some embodiments, the ATP-generating substrate is capable of reacting with the ADP to generate the ATP under the catalysis of the ATP-generating enzyme.

### Beneficial technical effects of the present disclosure

The present disclosure provides a method capable of consistently keeping sequencing speed stable during the sequencing process, and further maintaining high throughput and high accuracy of sequencing. Compared with other methods (for example, overall change of the sequencing buffer, or addition of ATP), the method and the kit of the present disclosure can keep the concentration of ATP relatively constant during the sequencing process, so that the sequencing rate can be better kept stable and unchanged.

### BRIEF DESCRIPTION OF DRAWINGS

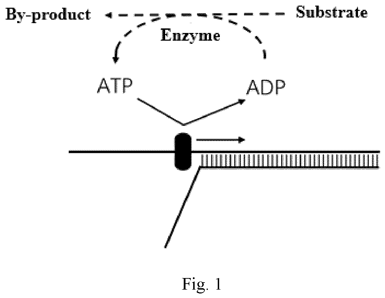

Fig. 1 shows a schematic diagram of the principle of the method of the present disclosure.
Fig. 2 shows the effect of ADP addition on the sequencing rate, wherein Fig. 2A is the result of a control group (no ADP added), Fig. 2B is the result of an experimental group 1 (added with 15 mM ADP), and Fig. 2C is the result of an experimental group 2 (added with 30 mM ADP).
Fig. 3 shows the effect of the control group and the experimental group 1 (containing PEP and PK) on the sequencing rate in Example 2, wherein Fig. 3A is the attenuation result of the sequencing rate and Fig. 3B is the change of sequencing rate percentage over time (an initial rate is 100%).
Fig. 4 shows the effect of the experimental group 2 (containing PEP and PK) on the sequencing rate in Example 2, wherein Fig. 4A is the attenuation result of the sequencing rate and Fig. 4B is the change of sequencing rate percentage over time (an initial rate is 100%).
Fig. 5 shows the effect of the experimental group 3 (containing PEP and PK) on the sequencing rate in Example 2, wherein Fig. 5A is the attenuation result of the sequencing rate and Fig. 5B is the change of sequencing rate percentage over time (an initial rate is 100%).
Fig. 6 shows the effect of the control group and the experimental group (containing Ac and AcK) on the sequencing rate in Example 3, wherein Fig. 6A is the attenuation result of the sequencing rate and Fig. 6B is the change of sequencing rate percentage over time (an initial rate is 100%).
Fig. 7 shows the impact of the control group and the experimental group (containing Cre and CK) on the sequencing rate in Example 4, wherein Fig. 7A is the attenuation result of the sequencing rate and Fig. 7B is the change of sequencing rate percentage over time (an initial rate is 100%).

### DESCRIPTION OF EMBODIMENTS

### Sequence information

The information on some of the sequences involved in the present disclosure is shown in Table 1 below.

**Table 1. Information on some of the sequences**

| SEQ ID NO: | Sequence Description | Sequence information |
|---|---|---|
| 1 | Amino acid sequence of wild type CsgG transmembra ne protein | CLTAPPKEAARPTLMPRAQSYKDLTHLPAPTGKIFVSVYNIQDETGQFK PYPASNFSTAVPQSATAMLVTALKDSRWFIPLERQGLQNLLNERKIIRAA QENGTVAINNRIPLQSLTAANIMVEGSIIGYESNVKSGGVGARYFGIGA DTQYQLDQIAVNLRVVNVSTGEILSSVNTSKTILSYEVQAGVFRFIDYQ RLLEGEVGYTSNEPVMLCLMSAIETGVIFLINDGIDRGLWDLQNKAER QNDILVKYRHMSVPPESSAWSHPQFEK |
| 2 | Wild type T4 Dda helicase | MTFDDLTEGQKNAFNIVMKAIKEKKHHVTINGPAGTGKTTLTKFIIEALIST GGTGIILAAPTHAAKKILSKLSGKEASTIHSILKINPVTYEENVLFEQKEVPD LAKCRVLICDEVSMYDRKLFKILLSTIPPWCTIIGIGDNKQIRPVEPGENTAY ISPFFTHKDFYQCELTEVKRSNAPIIDVATDVRNGKWNYDKVVDGHGVRG FTGDTALRDFMVNYFSIVKSLDDLFENRVMAFTNKSVDKLNSIIRKKIFETD KDFIVGEIIVMQEPLFKTYKIDGKPVSEIIFNNGQLVRIIEAEYTSTFVKARG VPGEYLIRHWDLTVETYGDDEYYREKIKIISSDEELYKFNLFLAKTAETYKN WNKGGKAPWSDFWDAKSQFSKVKALPASTFHKAQGMSVDRAFIYTPCIH YADVELAQQLLYVGVTRGRYDVFYV |
| 3 | pUC57 sequencing library | TCGCGCGTTTCGGTGATGACGGTGAAAACCTCTGACACATGCAGCTCCC GGAGACGGTCACAGCTTGTCTGTAAGCGGATGCCGGGAGCAGACAAGC CCGTCAGGGCGCGTCAGCGGGTGTTGGCGGGTGTCGGGGCTGGCTTAA CTATGCGGCATCAGAGCAGATTGTACTGAGAGTGCACCATATGCGGTGT GAAATACCGCACAGATGCGTAAGGAGAAAATACCGCATCAGGCGCCATT CGCCATTCAGGCTGCGCAACTGTTGGGAAGGGCGATCGGTGCGGGCCT CTTCGCTATTACGCCAGCTGGCGAAAGGGGGATGTGCTGCAAGGCGATT AAGTTGGGTAACGCCAGGGTTTTCCCAGTCACGACGTTGTAAAACGAC GGCCAGTGAATTCGAGCTCGGTACCTCGCGAATGCATCTAGATATCGGAT CCCGGGCCCGTCGACTGCAGAGGCCTGCATGCAAGCTTGGCGTAATCAT GGTCATAGCTGTTTCCTGTGTGAAATTGTTATCCGCTCACAATTCCACAC AACATACGAGCCGGAAGCATAAAGTGTAAAGCCTGGGGTGCCTAATGA GTGAGCTAACTCACATTAATTGCGTTGCGCTCACTGCCCGCTTTCCAGTC |
| | | GGGAAACCTGTCGTGCCAGCTGCATTAATGAATCGGCCAACGCGCGGG GAGAGGCGGTTTGCGTATTGGGCGCTCTTCCGCTTCCTCGCTCACTGAC TCGCTGCGCTCGGTCGTTCGGCTGCGGCGAGCGGTATCAGCTCACTCAA AGGCGGTAATACGGTTATCCACAGAATCAGGGGATAACGCAGGAAAGA ACATGTGAGCAAAAGGCCAGCAAAAGGCCAGGAACCGTAAAAAGGCC GCGTTGCTGGCGTTTTTCCATAGGCTCCGCCCCCCTGACGAGCATCACA AAAATCGACGCTCAAGTCAGAGGTGGCGAAACCCGACAGGACTATAAA GATACCAGGCGTTTCCCCCTGGAAGCTCCCTCGTGCGCTCTCCTGTTCC GACCCTGCCGCTTACCGGATACCTGTCCGCCTTTCTCCCTTCGGGAAGC GTGGCGCTTTCTCATAGCTCACGCTGTAGGTATCTCAGTTCGGTGTAGGT CGTTCGCTCCAAGCTGGGCTGTGTGCACGAACCCCCCGTTCAGCCCGA CCGCTGCGCCTTATCCGGTAACTATCGTCTTGAGTCCAACCCGGTAAGA CACGACTTATCGCCACTGGCAGCAGCCACTGGTAACAGGATTAGCAGAG CGAGGTATGTAGGCGGTGCTACAGAGTTCTTGAAGTGGTGGCCTAACTA CGGCTACACTAGAAGAACAGTATTTGGTATCTGCGCTCTGCTGAAGCCA GTTACCTTCGGAAAAAGAGTTGGTAGCTCTTGATCCGGCAAACAAACC ACCGCTGGTAGCGGTGGTTTTTTTGTTTGCAAGCAGCAGATTACGCGCA GAAAAAAAGGATCTCAAGAAGATCCTTTGATCTTTTCTACGGGGTCTGA CGCTCAGTGGAACGAAAACTCACGTTAAGGGATTTTGGTCATGAGATTA TCAAAAAGGATCTTCACCTAGATCCTTTTAAATTAAAAATGAAGTTTTAA ATCAATCTAAAGTATATATGAGTAAACTTGGTCTGACAGTTACCAATGCT TAATCAGTGAGGCACCTATCTCAGCGATCTGTCTATTTCGTTCATCCATAG TTGCCTGACTCCCCGTCGTGTAGATAACTACGATACGGGAGGGCTTACC ATCTGGCCCCAGTGCTGCAATGATACCGCGAGACCCACGCTCACCGGCT CCAGATTTATCAGCAATAAACCAGCCAGCCGGAAGGGCCGAGCGCAGA AGTGGTCCTGCAACTTTATCCGCCTCCATCCAGTCTATTAATTGTTGCCG GGAAGCTAGAGTAAGTAGTTCGCCAGTTAATAGTTTGCGCAACGTTGTT GCCATTGCTACAGGCATCGTGGTGTCACGCTCGTCGTTTGGTATGGCTTC ATTCAGCTCCGGTTCCCAACGATCAAGGCGAGTTACATGATCCCCCATGT TGTGCAAAAAAGCGGTTAGCTCCTTCGGTCCTCCGATCGTTGTCAGAAG TAAGTTGGCCGCAGTGTTATCACTCATGGTTATGGCAGCACTGCATAATT CTCTTACTGTCATGCCATCCGTAAGATGCTTTTCTGTGACTGGTGAGTAC TCAACCAAGTCATTCTGAGAATAGTGTATGCGGCGACCGAGTTGCTCTT GCCCGGCGTCAATACGGGATAATACCGCGCCACATAGCAGAACTTTAAA AGTGCTCATCATTGGAAAACGTTCTTCGGGGCGAAAACTCTCAAGGATC TTACCGCTGTTGAGATCCAGTTCGATGTAACCCACTCGTGCACCCAACT GATCTTCAGCATCTTTTACTTTCACCAGCGTTTCTGGGTGAGCAAAAAC AGGAAGGCAAAATGCCGCAAAAAAGGGAATAAGGGCGACACGGAAAT GTTGAATACTCATACTCTTCCTTTTTCAATATTATTGAAGCATTTATCAGG GTTATTGTCTCATGAGCGGATACATATTTGAATGTATTTAGAAAAATAAAC AAATAGGGGTTCCGCGCACATTTCCCCGAAAAGTGCCACCTGACGTCTA AGAAACCATTATTATCATGACATTAACCTATAAAAATAGGCGTATCACGA GGCCCTTTCGTC |

### Examples

The present disclosure is described by reference to the following examples which are intended to illustrate the present disclosure by examples (not to limit the present disclosure).

Unless otherwise specified, the experimental methods of molecular biology used in the present disclosure are performed basically with reference to the methods described in J. Sambrook et al., Molecular Cloning: Laboratory Manual, 2nd edition, Cold Spring Harbor Laboratory Press, 1989, and F. M. Ausubel et al., eds., Comprehensive Guide to Molecular Biology Experiments, 3rd edition, John Wiley & Sons, Inc., 1995. Those skilled in the art know that embodiments describe the present disclosure by way of examples and are not intended to limit the protection scope claimed by the present disclosure.

### Example 1

The inventors of the present disclosure have conducted multi-faceted explorations of the sequencing system and ultimately unexpectedly discovered that adding ADP to the sequencing buffer of the system would reduce the sequencing rate.

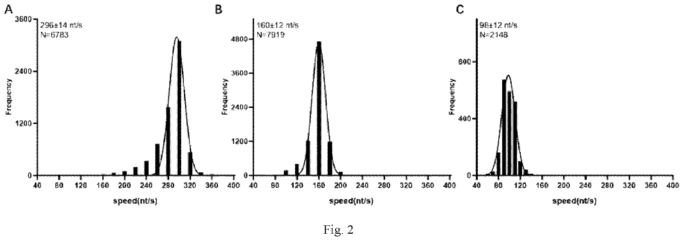

Specifically, a CsgG transmembrane protein mutant (the amino acid sequence of the wild type is shown in SEQ ID NO.1, and the mutation sites are: Y51A/F56Q/R97W/R192D) was used as a nanopore, and a nanopore detection method was used for detection. After a single CsgG transmembrane protein was inserted into the phospholipid bilayer, a buffer (470 mM KCl, 25 mM HEPES, and 1 mM EDTA, at PH 8.0) without fuel (ATP) was used to flow through the system to remove excess transmembrane protein. A constructed pUC57 sequencing library (SEQ ID NO.3) including T4 Dda helicase (the encoding amino acid sequence of the wild type is shown in SEQ ID NO.2, and the mutation sites are E94C/C109A/C136A/A360C) and the sequencing buffer were pre-mixed and then added to a nanopore experimental system. The sequencing of the CsgG transmembrane protein was detected at 0.18V voltage. Wherein:
in the control group (Fig. 2A), the sequencing buffer was 470 mM KCl, 25 mM HEPES, 30 mM ATP, 25 mM MgCl₂, and 1 mM EDTA, PH 8.0;
in experimental group 1 (Fig. 2B), the sequencing buffer was 470 mM KCl, 25 mM HEPES, 30 mM ATP, 15 mM ADP, 25 mM MgCl₂ and 1 mM EDTA, PH 8.0;
in experimental group 2 (Fig. 2C), the sequencing buffer was 470 mM KCl, 25 mM HEPES, 30 mM ATP, 30 mM ADP, 25 mM MgCl₂ and 1 mM EDTA, PH 8.0;

The experimental results are shown in Fig. 2. The abscissa of Fig. 2 is the sequencing speed and the ordinate is a frequency corresponding to the sequencing speed. It can be seen from Fig. 2 that adding ADP to the buffer of the sequencing system can reduce the sequencing speed compared with the control group. In addition, the sequencing rate of the experimental group 2 with larger addition amount of ADP is further decreased compared with that of the experimental group 1.

### Example 2

Based on the above example, the applicant has further explored the sequencing system, i.e., adding components to the sequencing buffer of the system to reduce the amount of ADP generated by the system during the sequencing process, while increasing the amount of ATP in the system during the sequencing process.

Specifically, a CsgG transmembrane protein mutant (the amino acid sequence of the wild type is shown in SEQ ID NO.1, and the mutation sites are: Y51A/F56Q/R97W/R192D) was used as a nanopore, and a nanopore detection method was used for detection. After a single CsgG transmembrane protein was inserted into the phospholipid bilayer, a buffer (470 mM KCl, 25 mM HEPES, and 1 mM EDTA, at PH 8.0) without fuel (ATP) was used to flow through the system to remove excess transmembrane protein. A constructed pUC57 sequencing library (SEQ ID NO.3) including T4 Dda helicase (the encoding amino acid sequence of the wild type is shown in SEQ ID NO.2, and the mutation sites are E94C/C109A/C136A/A360C) and the sequencing buffer were pre-mixed and then added to a nanopore experimental system. The sequencing of the CsgG transmembrane protein was detected at 0.18V voltage. Wherein:
in the control group, the sequencing buffer was 470 mM KCl, 25 mM HEPES, 5 mM ATP, 25 mM MgCl₂ and 1 mM EDTA, at PH 8.0.

Three concentrations were set in the experimental groups:
the sequencing buffer in the experimental group 1 was 470 mM KCl, 25 mM HEPES, 5 mM ATP, 25 mM MgCl₂, 1 mM EDTA, PH 8.0, 5 mM PEP (phosphoenolpyruvate, Roche, catalog No.: 10108294001), and 0.02 U/mL PK (pyruvate kinase, Roche, catalog No.: 10109045001);
the sequencing buffer in the experimental group 2 was 470 mM KCl, 25 mM HEPES, 5 mM ATP, 25 mM MgCl₂, 1 mM EDTA, PH 8.0, 5 mM PEP (phosphoenolpyruvate), and 0.2 U/mL PK (pyruvate kinase).
the sequencing buffer in the experimental group 3 was 470 mM KCl, 25 mM HEPES, 5 mM ATP, 25 mM MgCl₂, 1 mM EDTA, PH 8.0, 5 mM PEP (phosphoenolpyruvate), and 2 U/mL PK (pyruvate kinase).

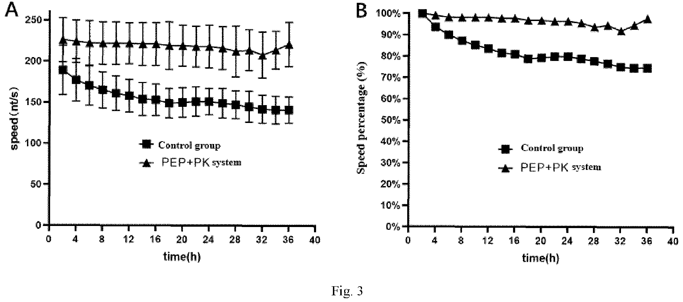

The experimental results of the experimental groups 1, 2 and 3 are shown in Figs. 3, 4 and 5, respectively. Compared with the control group, the sequencing method and the sequencing buffer of the present disclosure were significantly more stable during the sequencing process, and in the later stage of sequencing, the sequencing rate was almost 20% higher than that of the control group. During the sequencing process, the sequencing speed of the present disclosure was hardly reduced.

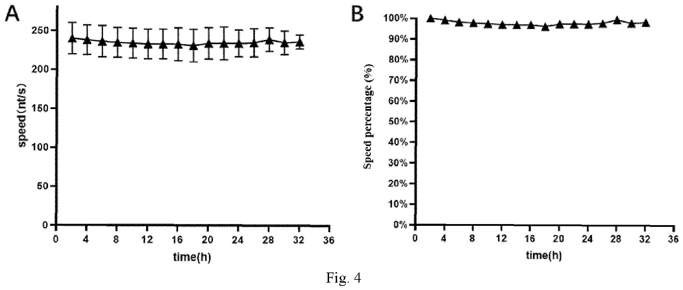

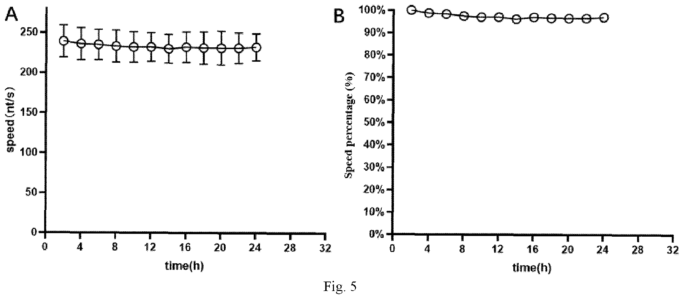

Moreover, the experimental results in Fig. 4 and Fig. 5 show that the change of the concentration of PEP or PK (pyruvate kinase) in the system does not affect the sequencing rate, and the sequencing speed has no obvious attenuation.

### Example 3

The experimental conditions and steps of the present disclosure are the same as those of Example 2, except that:
in the control group, the formula of the sequencing buffer was 470 mM KCl, 25 mM HEPES, 5 mM ATP, 25 mM MgCl₂, and 1 mM EDTA, PH 8.0;
in the experimental group, the formula of the sequencing buffer was 470mM KCl, 25 mM HEPES, 5 mM ATP, 25 mM MgCl₂, 1 mM EDTA, PH 8.0, 5 mM Ac (lithium potassium acetyl phosphate, sigma, 01409-500MG), and 0.02 U/mL AcK (acetate kinase, sigma, A7437-250UN).

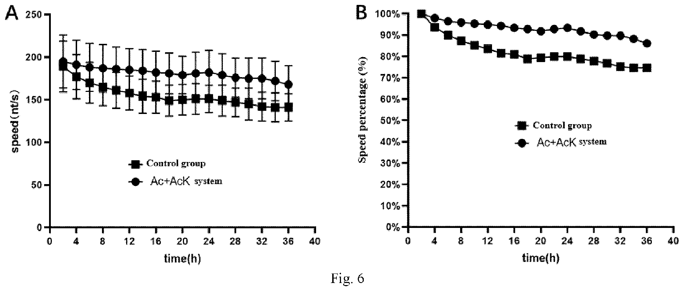

The experimental results (Fig. 6) show that compared with the control group, the sequencing method and the sequencing buffer of the present disclosure were significantly more stable during the sequencing process, and in the later stage of sequencing, the sequencing rate was almost 20% higher than that of the control group.

### Example 4

The experimental conditions and steps of the present disclosure are the same as those of Example 2, except that:
in the control group, the formula of the sequencing buffer was 470 mM KCl, 25 mM HEPES, 5 mM ATP, 25 mM MgCl₂, and 1 mM EDTA, PH 8.0;
in the experimental group, the formula of the sequencing buffer was 470 mM KCl, 25 mM HEPES, 5 mM ATP, 25 mM MgCl₂, 1 mM EDTA, PH 8.0, and 5 mM Cre (creatine phosphate disodium, sigma, P7936-1G) /0.02 U/mL CK (creatine phosphokinase, sigma, C3755-3.5KU).

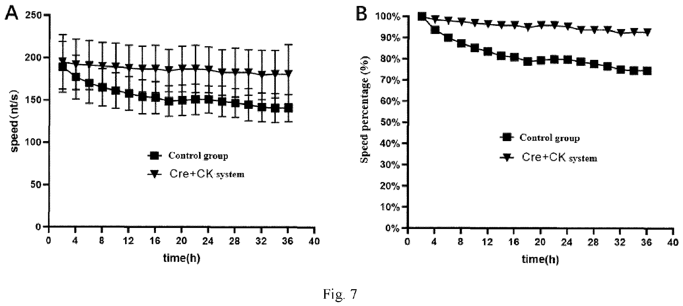

The experimental results (Fig. 7) show that compared with the control group, the sequencing method and the sequencing buffer of the present disclosure were significantly more stable during the sequencing process, and in the later stage of sequencing, the sequencing rate was almost 20% higher than that of the control group. During the sequencing process, the sequencing speed of the present disclosure was hardly reduced.

Although the specific embodiments of the present disclosure have been described in detail, those skilled in the art will understand that various modifications and changes may be made to the details based on all the teachings published, and these changes are all within the protection scope of the present disclosure. The entire scope of the present disclosure is defined by the appended claims and any equivalents thereof.

## Claims

1. A method for sequencing a double-stranded target polynucleotide, comprising:
(a) providing a double-stranded target polynucleotide, a membrane containing a transmembrane pore, a helicase, an ATP-generating enzyme, and an ATP-generating substrate, wherein the ATP-generating substrate is capable of reacting with ADP to generate ATP under the catalysis of the ATP-generating enzyme;
(b) contacting the double-stranded target polynucleotide with the transmembrane pore, the helicase, the ATP-generating enzyme, and the ATP-generating substrate, wherein double strands of the double-stranded target polynucleotide are separated by the helicase to form a single-stranded target polynucleotide, and wherein the helicase enables the single-stranded polynucleotide to move in the transmembrane pore, allowing a portion of nucleotides in the single-stranded polynucleotide to interact with the transmembrane pore; and
(c) measuring current that passes through the transmembrane pore during each interaction to determine the sequence of the double-stranded target polynucleotide.

2. The method according to claim 1, having one or more features selected from the following:
(1) the ATP-generating enzyme is selected from pyruvate kinase, acetate kinase, creatine phosphokinase, serine kinase, threonine kinase, tyrosine kinase, FoF1-ATPase, polyphosphate kinase, nucleoside diphosphate kinase, or any combination thereof;
(2) the ATP-generating enzyme is a pyruvate kinase, and the ATP-generating substrate is phosphoenolpyruvate;
(3) the ATP-generating enzyme is acetate kinase, and the ATP-generating substrate is lithium potassium acetyl phosphate;
(4) the ATP-generating enzyme is creatine phosphokinase, and the ATP-generating substrate is creatine phosphate disodium;
(5) the ATP-generating enzyme is FoF1-ATPase, and the ATP-generating substrate is inorganic phosphate;
(6) the ATP-generating enzyme is creatine phosphokinase, and the ATP-generating substrate is creatine phosphate;
(7) the ATP-generating enzyme is polyphosphate kinase, and the ATP-generating substrate is polyphosphate; and
(8) the ATP-generating enzyme is nucleoside diphosphate kinase, and the ATP-generating substrate is nucleoside triphosphate.

3. The method according to claim 1 or 2, wherein the helicase has one or more features selected from the following:
(1) the helicase is selected from Dda, UvrD, Rep, RecQ, PcrA, eIF4A, NS3, Rep, gp41, T7gp4, or any combination thereof;
(2) the helicase is further linked to an additional polypeptide, the additional polypeptide being selected from a label, an enzyme cleavage site, a signal peptide or leading peptide, a detectable marker, or any combination thereof; and
(3) the helicase is a wild type Dda or a mutant thereof; and preferably, the helicase has an amino acid sequence as set forth in SEQ ID NO: 2.

4. The method according to any one of claims 1 to 3, wherein the transmembrane pore has one or more features selected from the following:
(1) the transmembrane pore is a transmembrane protein pore or a transmembrane solid-state pore; and preferably, the transmembrane protein pore is selected from hemolysin, MspA, MspB, MspC, MspD, Frac, ClyA, PA63, CsgG, CsgD, XcpQ, SP1, phi29 connector, T7 connector, GspD, InvG, or any combination thereof;
(2) the transmembrane pore is further linked to an additional polypeptide, the additional polypeptide being selected from a label, an enzyme cleavage site, a signal peptide or leading peptide, a detectable marker, or any combination thereof; and
(3) the transmembrane pore is a wild type CsgG protein or a mutant thereof; and preferably, the transmembrane pore has an amino acid sequence as set forth in SEQ ID NO: 1.

5. The method according to any one of claims 1 to 4, wherein the membrane is an amphiphilic layer (e.g., phospholipid bilayer) or a polymer membrane (e.g., di-block and tri-block).

6. The method according to any one of claims 1 to 5, wherein the double-stranded target polynucleotide has one or more features selected from the following:
(1) the double-stranded target polynucleotide is a double-stranded DNA and/or a double-stranded DNA-RNA hybrid;
(2) the double-stranded target polynucleotide is naturally occurring and/or artificially synthesized;
(3) the double-stranded target polynucleotide is obtained from biological samples, the biological samples being extracted from viruses, prokaryotes (e.g., bacteria), eukaryotes (e.g., plants (e.g., cereals, legumes, fruits, or vegetables)), mammals (e.g., horses, cattle, mice or humans), or any combination thereof;
(4) the double strands of the double-stranded target polynucleotide are linked by a bridging part at or near one end of the target polynucleotide, the bridging part being selected from a polymer linker, a chemical linker, a polynucleotide, or a polypeptide; and
(5) the double-stranded target polynucleotide is circular or linear.

7. The method according to any one of claims 1 to 6, having one or more features selected from the following:
(1) the double-stranded target polynucleotide comprises at least one single-stranded overhang (e.g., a 5' overhang and/or a 3' overhang), the single-stranded overhang comprising a leader sequence configured to guide a nucleic acid strand linked thereto into the pore;
(2) in step (b), the double-stranded target polynucleotide is contacted with the helicase to form a complex, and the complex is contacted with the transmembrane pore; and
(3) in step (b), a reagent for nanopore sequencing is contacted with the helicase; preferably, the reagent for nanopore sequencing is selected from ATP, an inorganic salt (e.g., chloride salt, e.g., sodium chloride, potassium chloride or lithium chloride), a buffer (HEPES and/or Tris-HCl), EDTA, metal ions (e.g., Mn²⁺, Mg²⁺, Co⁺, Zn²⁺, Cu²⁺, Cu⁺, Ni⁺, Fe²⁺ or Fe³⁺), or any combination thereof.

8. A kit, comprising a membrane containing a transmembrane pore, a helicase, an ATP-generating enzyme, and an ATP-generating substrate, optionally, the kit further comprising a reagent for nanopore sequencing,
preferably, the reagent for nanopore sequencing is selected from ATP, an inorganic salt (e.g., chloride salt, e.g., sodium chloride, potassium chloride or lithium chloride), a buffer (HEPES and/or Tris-HCl), EDTA, metal ions (e.g., Mn²⁺, Mg²⁺, Co⁺, Zn²⁺, Cu²⁺, Cu⁺, Ni⁺, Fe²⁺ or Fe³⁺), or any combination thereof;
preferably, the ATP-generating enzyme is selected from pyruvate kinase, acetate kinase, creatine phosphokinase, serine kinase, threonine kinase, tyrosine kinase, FoF1-ATPase, polyphosphate kinase, nucleoside diphosphate kinase, or any combination thereof;
preferably, the kit comprises: a membrane containing a transmembrane pore; a helicase; an ATP; (I) pyruvate kinase and phosphoenolpyruvate, (II) acetate kinase and lithium potassium acetyl phosphate, (III) creatine phosphokinase and creatine phosphate, (IV) FoF1-ATPase and inorganic phosphate, (V) creatine phosphokinase and creatine phosphate; (VI) polyphokinase and polyphosphate, (VII) nucleoside diphosphate kinase and nucleoside triphosphate, or any combination of (I) to (VII); and
preferably, the kit is used to sequence a double-stranded target polynucleotide.

9. The kit according to claim 8, having one or more features selected from the following:
(1) the helicase is selected from Dda, UvrD, Rep, RecQ, PcrA, eIF4A, NS3, Rep, gp41, T7gp4, or any combination thereof;
(2) the helicase is further linked to an additional polypeptide, the additional polypeptide being selected from a label, an enzyme cleavage site, a signal peptide or leading peptide, a detectable marker, or any combination thereof;
(3) the helicase is a wild type Dda or a mutant thereof; and preferably, the helicase has an amino acid sequence as set forth in SEQ ID NO: 2;
(4) the transmembrane pore is a transmembrane protein pore or a transmembrane solid-state pore; and preferably, the transmembrane protein pore is selected from hemolysin, MspA, MspB, MspC, MspD, Frac, ClyA, PA63, CsgG, CsgD, XcpQ, SP1, phi29 connector, T7 connector, GspD, InvG, or any combination thereof;
(5) the transmembrane pore is further linked to an additional polypeptide, the additional polypeptide being selected from a label, an enzyme cleavage site, a signal peptide or leading peptide, a detectable marker, or any combination thereof;
(6) the transmembrane pore is a wild type CsgG protein or a mutant thereof; and preferably, the transmembrane pore has an amino acid sequence as set forth in SEQ ID NO: 1; and
(7) the membrane is an amphiphilic layer (e.g., phospholipid bilayer) or a polymer membrane (e.g., di-block and tri-block).

10. Use of the kit according to claim 8 or 9 in the preparation of a sequencing device for sequencing a double-stranded target polynucleotide.
